# EUROPEAN PATENT SPECIFICATION

(11) **EP 2 862 566 B1**
(45) Date of publication and mention of the grant of the patent: **09.03.2022**
(21) Application number: 14194194.8
(22) Date of filing: 31.01.2012
(51) Int. Cl.: A61P 1/00, A61P 1/10, A61P 43/00, A61K 9/08, A61K 9/00, A61K 31/77, A61K 33/00

(54) **Improvements in and relating to compositions**
Verbesserungen an und im Zusammenhang mit Zusammensetzungen
Améliorations apportées et relatives à des compositions

(30) Priority: 31.01.2011 GB 201101666; 01.02.2011 GB 201101738; 13.01.2012 GB 201200637
(43) Date of publication of application: 22.04.2015
(62) Divisional of application: 12702615.1
(73) Proprietor: Norgine Healthcare B.V., 1083 HP Amsterdam (NL)
(72) Inventor: Morrissey, Frances, Hengoed, Mid-Glamorgan CF82 8SJ (GB); Padfield, Dawn, Hengoed, Mid-Glamorgan CF82 8SJ (GB); Seldon, Chris, Rhondda, CF39 0EA (GB)
(74) Representative: Abel & Imray LLP

(56) References cited:
- WO-A1-2005/049049
- WO-A1-2011/012866
- GB-A- 2 471 954
- US-A1- 2005 003 021
- None
- Constantinos M. Paleos ET AL: "Interaction between complementary liposomes: a process leading to multicompartment systems formation", Journal of Molecular Recognition, vol. 19, no. 1, 1 January 2006 (2006-01-01), pages 60-67, XP055174901, ISSN: 0952-3499, DOI: 10.1002/jmr.758

## Description

The present invention relates to solutions that find utility in the treatment of constipation and/or faecal impaction. In particular, it relates to the use of ready-to-use solutions comprising polyethylene glycol (PEG) and electrolytes for the treatment of constipation and/or faecal impaction.

Constipation is a widespread condition which generally gives rise to discomfort. The physical presence of faeces retained in the colon and/or the rectum gives rise to a feeling of malaise and headaches. In extreme cases of prolonged constipation, dyschezia may result from the presence of scybala or faecaliths in the rectum.

Numerous treatments for constipation have been developed, including dietary manipulation (e.g. increasing the fibre content of the diet and removing foods considered to cause constipation), laxatives and enemas. Laxatives are agents that promote and assist defecation. Osmotic laxatives act to retain water in the colonic lumen, thereby counteracting the normal dehydrating action of the colon. By suppressing the dehydration action of the colon, the osmotic laxative produces a faecal stream which is softer, bulkier and easier to expel.

A number of osmotic laxative treatments currently in use comprise polyethylene glycol (PEG) and electrolytes. Various such PEG / electrolyte products are on the market in many countries. An example of such a product is MOVICOL (registered trademark of Edra AG, exclusively licensed to the Norgine group of companies, and marketed in the UK by Norgine Limited, Chaplin House, Widewater Place, Moorhall Road, Harefield, Uxbridge, Middlesex UB9 6NS, United Kingdom). MOVICOL is provided in a sachet containing 13.8g powder for making up into an oral solution. Each sachet contains: 13.1250g Macrogol 3350, 0.3507g sodium chloride, 0.1785g sodium bicarbonate and 0.0466g potassium chloride. This is the standard dose of MOVICOL. It also contains flavouring and sweetener. MOVICOL has been on the market since 1995. MOVICOL PLAIN is essentially the same as MOVICOL but it does not contain flavouring or sweetener, so to adjust for the potassium content of the sweetener, it contains slightly more potassium chloride. Each sachet of MOVICOL PLAIN contains: 13.1250g Macrogol 3350, 0.3508g sodium chloride, 0.1786g sodium bicarbonate and 0.0502g potassium chloride. When MOVICOL or MOVICOL PLAIN is made into a drink with water to a volume of 125 millilitres, each sachet gives the equivalent of: 65 millimoles/litre sodium, 53 millimoles/litre chloride, 17 millimoles/litre bicarbonate and 5.4 millimoles/litre potassium.

One standard dose of MOVICOL is provided as a unit treatment in powder form in one sachet. Patients are advised to combine the powder contents of a sachet with water to make up a drink of 125ml. It is found that dissolution can, in practice, take some time. It is important with a solution of the MOVICOL type that the patient does not to attempt to speed the dissolution by heating as that will lead to decomposition of the bicarbonate component. The time taken for the powder to dissolve causes inefficiency in the care-home or hospital setting where solutions are prepared by professional care-providers. In the self-administration domestic setting, it can cause frustration in the patient and risks the patient taking an incompletely dissolved preparation, which would reduce the efficacy of the treatment. The sachets are made of a laminate consisting of four layers: low density polyethylene, aluminium, low density polyethylene and paper. Some patients have difficulties manipulating and tearing open the sachets. The sachets are not reusable.

For the treatment of constipation, patients are advised to take one sachet dissolved to 125ml 1-3 times a day, according to the severity of the constipation. Treatment with MOVICOL usually lasts about 2 weeks. There are various situations in which MOVICOL is recommended for longer treatments than 2 weeks, particularly in patients who take drugs that cause constipation (e.g. opioids, such as morphine) or have a disease that has associated constipation (for example Parkinson's disease or multiple sclerosis (MS)). Usually, for long term treatment in such chronic treatment situations, the number of doses per day can be adjusted down to either one or two.

For the treatment of faecal impaction, the recommended treatment is 8 sachets a day (each dissolved to 125ml), taken within 6 hours. That number of doses may be needed for up to 3 days.

Therefore, MOVICOL and other PEG-based laxatives generally require the user to make up a solution for consumption by combining a dry powder with water. This is generally inconvenient, particularly for the elderly, time consuming and increases the possibility that the instructions, typically provided with such products, are not followed as directed. In addition, it requires the user to have drinkable water or other aqueous solution available.

WO2005/049049 purports to describe a polyethylene glycol liquid concentrate for treatment of constipation or for gastrointestinal lavage that is chemically stable and resistant to microbial contamination.

US2005/003021 purports to describe a bowel cleansing preparation which is useful in the pretreatment prior to colonoscopy or colonic surgery containing a water-soluble polymer and electrolytes of 30-150 mEq/L of sodium ion, 3-20 mEq/L of potassium ion, and 10-30 mEq/L of bicarbonate ion, wherein 0.1-3 g/dL of magnesium ion as magnesium element is contained, and polyethylene glycol having an average molecular weight of about 7,300 to about 9,300 is used as the water-soluble polymer.

GB2471954 purports to describe a dry composition for admixture with water, per litre of solution to be made, the following components:(a) 85 to 115 g polyethylene glycol (PEG) having an average molecular weight of 2500 to 4500;(b) 6 to 9 g sodium sulphate;(c) 2 to 3 g sodium chloride;(d) 0.5 to 1.5g potassium chloride;(e) 5 to 15g of an organic acid component; and (f) orange flavouring, wherein the composition is preferably presented in two or more parts. GB2471954 also purports to describe solutions, kits, unit doses and methods that comprise or use the compositions. The compositions of GB2471954 are said to preferably be used to clean the colon and gastrointestinal tract. GB2471954 also purports to describe a dry powder composition comprising PEG and orange flavouring which is substantially free from glucose and is freeflowing after 3 months at 25°C and 60% relative humidity.

It is an object of the present invention to provide a PEG-based laxative product that is easy and convenient to take, is efficacious, has an acceptable taste and an acceptable shelf-life.

The present invention provides an aqueous solution single unit dose amount for use according to claim 1.

The present invention further provides a kit as claimed in claim 8.

The present invention further provides a kit for use according to claim 12.

Also described herein, but not claimed, is a method of treating constipation and/or faecal impaction in a mammalian subject, such as a human, comprising orally administering, without further dilution, an aqueous solution, preferably substantially free of sulphate, comprising (or consisting essentially of) the following components:
(a) 350 to 600g/L PEG having an average molecular weight of 2500 to 4500 Da;
(b) 8.0 to 20 g/L sodium chloride;
(c) 1.0 to 3.0 g/L potassium chloride;
(d) 3.0 to 11 g/L sodium bicarbonate;
(e) optional preservative;
(f) optional sweetener; and
(g) optional flavouring.

Solutions described herein find use, in particular, in a method of treating constipation comprising orally administering an aqueous solution as described *supra.* In another aspect of the present invention, there is provided an aqueous solution for use as described *supra* for oral administration, without further dilution, for the treatment of constipation and/or faecal impaction. The solution is especially suitable for the treatment of constipation.

By "without further dilution" is meant that the aqueous solutions for use according to the present invention are "ready-to-use", that is, neither do they require the combination of a dry powder with water nor do they require combination with water or any other liquid (i.e. they do not require any dilution) prior to consumption. It is to be understood that there may be an insubstantial amount of dilution by the saliva or dregs of liquid (e.g. from rinsing a glass or other drinking vessel) upon administration/consumption, but this is not to be taken as "further dilution" within the context of this invention. In particular, "without further dilution" means that the solutions for use according to the invention are used/administered without being combined with added liquid, such as water.

As such, solutions for use according to the invention are particularly convenient to administer/consume. This is particularly advantageous for subjects that are frail (for example the geriatric population) where it is generally inconvenient to have to follow preparation instructions prior to consuming a product.

Solutions for use according to the invention are preferably substantially free from any sulphate component. In particular, solutions for use according to the invention are preferably substantially free from sodium sulphate. In this context, "substantially free from any sulphate component" is taken to mean free from any added sulphate component. Negligible amounts of sulphate salts may be present in other added components, or in the water that is used in the solutions. Such amounts are not substantial in this context.

Also described herein, but not claimed, is an aqueous solution for use in the treatment of constipation and/or faecal impaction by oral administration without further dilution, preferably substantially free of sulphate, comprising (or consisting essentially of) the following components:
(a) 350 to 600g/L PEG having an average molecular weight of 2500 to 4500 Da;
(b) 8.0 to 20 g/L sodium chloride;
(c) 1.0 to 3.0 g/L potassium chloride;
(d) 3.0 to 11 g/L sodium bicarbonate;
(e) 1.0 to 5.9 g/L (for example 3.0 to 5.9 g/L) of preservative;
(f) optional sweetener; and
(g) optional flavouring.

Also described herein, but not claimed, is an aqueous solution, preferably substantially free of sulphate, comprising (or consisting essentially of) the following components:
(a) 350 to 600g/L PEG having an average molecular weight of 2500 to 4500 Da;
(b) 8.0 to 20 g/L sodium chloride;
(c) 1.0 to 3.0 g/L potassium chloride;
(d) 3.0 to 11 g/L sodium bicarbonate;
(e) 1.0 to 5.9 g/L (for example 3.0 to 5.9 g/L) of preservative;
(f) 0.1 to 1.5 g/L sweetener; and
(g) optional flavouring.

Also described herein, but not claimed, is an aqueous solution, preferably substantially free of sulphate, comprising (or consisting essentially of) the following components:
(a) 350 to 600g/L PEG having an average molecular weight of 2500 to 4500 Da;
(b) 8.0 to 20 g/L sodium chloride;
(c) 1.0 to 3.0 g/L potassium chloride;
(d) 3.0 to 11 g/L sodium bicarbonate;
(e) 1.0 to 5.9 g/L (for example 3.0 to 5.9 g/L) of preservative;
(f) 0.1 to 1.5 g/L sweetener; and
(g) 1.0 to 10.0 g/L flavouring.

The term "thereabout" used throughout this specification is intended to convey that strict compliance with the indicated amount is not necessary and is intended to encompass insignificant variations e.g. due to manufacturing tolerances.

### Polyethylene glycol

The polyethylene glycol (PEG) used in solutions for use according to the invention has an average molecular weight (for example a weight average molecular weight), of 2500Da to 4500Da. The PEG may have an average molecular weight of 3000 to 4000. For example, the PEG may be PEG 3350 or PEG 4000 as defined in national pharmacopoeias. Further examples of suitable PEGs recognized in some national pharmacopoeias include Macrogols, for example Macrogol 4000. Optionally, the PEG used in compositions for use according to the invention may comprise two or more different PEG compounds.

Depending on the molecular weight of the PEG in a solution for use according to the invention, the upper limit of concentration of the PEG may be limited by the water solubility of the PEG.

Solutions for use according to the invention therefore comprise PEG in an amount of 350 to 600 g per litre, preferably within a range wherein the lower limit is 400, 450 or 500 g per litre and the upper limit is, independently, 600, 575 or 550 g per litre; for example 500 to 550 g per litre. For example a solution for use according to the invention may comprise 525 g of PEG per litre, for example 525 g of PEG 3350 per litre.

### Sodium Chloride

Solutions for use according to the invention preferably comprise sodium chloride in an amount of 8.0 to 20g per litre of solution, preferably within a range wherein the lower limit is 10, 11, 12 or 13 g per litre and the upper limit is, independently, 18, 17, 16 or 15 g per litre; for example 13 to 15 g per litre. For example a solution for use according to the invention may comprise approximately 14g of sodium chloride per litre, for example 14.028g per litre.

### Potassium Chloride

Solutions for use according to the invention preferably comprise potassium chloride in an amount of 1.0 to 3.0 g per litre, preferably within a range wherein the lower limit is 1.2, 1.4, 1.6, 1.7 or 1.8 g per litre and the upper limit is, independently, 2.7, 2.5, 2.3, 2.1 or 2.0 g per litre; for example 1.6 to 2.1 g per litre, for example 1.8 to 1.9g per litre. For example a solution for use according to the invention may comprise 1.864 g of potassium chloride per litre. An alternative solution for use according to the invention may comprise 1.268g or 2.008 g of potassium chloride per litre. In an embodiment, the potassium ion content may be provided by a potassium salt other than potassium chloride.

### Sodium bicarbonate

Solutions for use according to the invention preferably comprise sodium bicarbonate (also known as sodium hydrogen carbonate) in an amount of 3.0 to 11g per litre, preferably within a range wherein the lower limit is 5.0, 6.0, 6.5 or 7.0g per litre and the upper limit is, independently, 10, 9.0, 8.0 or 7.5g per litre; for example 6.5 to 8.0 g per litre. For example a solution for use according to the invention may comprise approximately 7.1 g per litre, for example 7.140g per litre.

In a solution for use according to the invention, the weight ratio of the components PEG, sodium chloride, potassium chloride and sodium bicarbonate is preferably approximately 13.125(PEG) : 0.3507 (NaCl) : 0.0466 (KCl) : 0.1785 (NaHCO₃), ie approximately 282 (PEG) : 7.5 (NaCl) : 1 (KCl) : 3.8 (NaHCO₃). For example it may be within the ranges 250 to 450 (PEG) : 5 to 15 (NaCl) : 1 (KCl) : 3 to 7.5 (NaHCO₃), for example 250 to 300 (PEG) : 5 to 10 (NaCl) : 1 (KCl) : 3 to 5 (NaHCO₃), for example within the ranges 275 to 285 (PEG) : 7 to 8 (NaCl) : 1 (KCl) : 3.6 to 4.0 (NaHCO₃).

In another embodiment of a solution for use according to the invention, the molar ratio of the individual ions in the components sodium chloride, potassium chloride and sodium bicarbonate is preferably approximately 65(Na⁺) : 53 (Cl⁻) : 5.0 (K⁺) : 17 (HCO₃⁻), ie approximately 13 (Na⁺) :10.6 (Cl⁻) : 1 (K⁺) : 3.4 (HCO₃⁻), For example it may be within the ranges 11 to 15 (Na⁺) : 8 to 13 (Cl⁻) : 1 (K⁺) : 2.8 to 4.0 (HCO₃⁻), for example within the ranges 12 to 14 (Na⁺) : 9 to 11 (Cl⁻) : 1 (K⁺) : 3.2 to 3.6 (HCO₃⁻).

Solutions for use according to the invention preferably comprise sodium present as sodium ions in an amount of 173 to 473 mmol per litre, preferably within a range wherein the lower limit is 231, 259, 282 or 305 mmol per litre and the upper limit is, independently, 427, 398, 369 or 345 mmol per litre; for example 305 to 345 mmol per litre. For example, a solution for use according to the invention may comprise approximately 325 mmol sodium present as sodium ions per litre. In one embodiment, solutions for use according to the invention preferably comprise potassium present as potassium ions in an amount of 14 to 43 mmol per litre, preferably within a range wherein the lower limit is 17, 23 or 26 mmol per litre and the upper limit is, independently, 39, 34 or 29 mmol per litre; for example from 26 to 29 mmol per litre. For example a solution for use according to the invention may comprise approximately 27 mmol potassium present as potassium ions per litre.

In another embodiment, solutions for use according to the invention preferably comprise potassium present as potassium ions in an amount of 13 to 40 mmol per litre, preferably within a range wherein the lower limit is 16, 19, 21, 23 or 24 mmol per litre and the upper limit is, independently, 36, 34, 31, 28 or 27 mmol per litre; for example 21 to 28 mmol per litre, for example from 24 to 25 mmol per litre. For example a solution for use according to the invention may comprise approximately 25 mmol potassium present as potassium ions per litre. Solutions for use according to the invention preferably comprise chloride ions in an amount of 150 to 382 mmol per litre, preferably within a range wherein the lower limit is 187, 207, 226 or 245 mmol per litre and the upper limit is, independently, 344, 325, 305 or 284 mmol per litre; for example from 246 to 281 mmol per litre. For example a solution for use according to the invention may comprise approximately 265 mmol chloride ions per litre.

Solutions for use according to the invention preferably comprise bicarbonate ions in an amount of 36 to 131 mmol per litre, preferably within a range wherein the lower limit is 60, 71, 77 or 83 mmol per litre and the upper limit is, independently, 119, 107, 95 or 89 mmol per litre; for example 77 to 95 mmol per litre. For example a solution for use according to the invention may comprise approximately 85 mmol per litre.

### Sweeteners

Solutions for use according to the invention may comprise one or more sweeteners. Preferred sweeteners include aspartame, acesulfame potassium (acesulfame K), sucralose and saccharine and combinations thereof. For example, solutions of the invention may comprise one or both of sucralose and acesulfame potassium (acesulfame K). Typically, a sweetener may be present at a level of for example 0.1 to 1.5g per litre such as 0.3 to 1.2g per litre, e.g. 0.5g to 1g per litre. Solutions for use according to may include a sweetener at a level of 0.7 to 1.5g per litre. Solutions for use according to may include a sweetener, for example at a level of 0.6 to 0.9g per litre, for example 0.70 to 0.95g per litre, for example 0.80 to 0.95g per litre. In one embodiment, the sweetener is at a level of 1.0g or thereabout per litre. In another embodiment, the sweetener is at a level of 0.85g or thereabout per litre.

In an embodiment, acesulfame K is present in an amount of, 0.20 to 0.60 g per litre, preferably within in a range in which the lower limit is 0.20, 0.30 or 0.35 g per litre and the upper limit is, independently, 0.60, 0.50 or 0.45 g per litre. For example, a solution for use according to the invention may comprise 0.40 g acesulfame K per litre.

In one embodiment, solutions for use according to the invention preferably comprise acesulfame ions in an amount of 1.0 to 3.0 mmol per litre, preferably within a range in which the lower limit is 1.0, 1.5 or 1.7 mmol per litre and the upper limit is, independently, 3.0, 2.5 or 2.2 mmol per litre. For example, a solution for use according to the invention may comprise approximately 2.0 mmol acesulfame ions per litre.

Also disclosed herein, but not claimed, is an aqueous solution comprising the following components at the following concentrations:
(a) 78 to 240 mmol/L PEG having an average molecular weight of 2500 to 4500 Da;
(b1) 173 to 473 mmol/L sodium present as sodium ions;
(c) 14 to 43 mmol/L potassium present as potassium ions;
(b2) 150 to 382 mmol/L chloride ions;
(d) 36 to 131 mmol/L bicarbonate ions;
(e) preservative;
(f1) 1.0 to 3.0 mmol/L acesulfame ions;
(f2) optional additional sweetener; and
(g) optional flavouring.

In one embodiment of a solution for use according to the invention, the molar ratio of the individual ions in the components sodium chloride, potassium chloride, sodium bicarbonate and acesulfame K is preferably approximately 65(Na⁺) : 53 (Cl⁻) : 5.4 (K⁺) : 17 (HCO₃⁻), ie approximately 12 (Na⁺) :10 (Cl⁻) : 1 (K⁺) : 3 (HCO₃⁻), For example it may be within the ranges 10 to 14 (Na⁺) : 8 to 12 (Cl⁻) : 1 (K⁺) : 2.5 to 3.7 (HCO₃⁻), for example within the ranges 11 to 13 (Na⁺) : 9 to 11 (Cl⁻) : 1 (K⁺) : 2.9 to 3.3 (HCO₃⁻).

In an embodiment, sucralose is present in an amount of 0.2 to 1.0g per litre, more preferably within in a range in which the lower limit is , 0.2 or 0.4 g per litre and the upper limit is, independently, 1.0, 0.8, 0.6, 0.5, 0.45, 0.3g per litre. For example, sucralose may be present in an amount of from 0.20 to 0.55g per litre, for example 0.30 to 0.55g per litre, for example 0.40 to 0.50g per litre. For example, a solution for use according to the invention may comprise 0.6 g or 0.45g sucralose per litre.

In solutions described herein, but not claimed, the preservative and sweetener are present in a weight ratio of preservative: sweetener of 1:1 to 7:1, preferably 1:1 to 5:1, more preferably 1:1 to 2:1.

In one embodiment, the solution for use according to the invention comprises both sucralose (as the optional additional sweetener) and acesulfame K. In this embodiment, acesulfame K is present in an amount of, 0.20 to 0.60 g per litre, preferably within a range in which the lower limit is 0.20, 0.30 or 0.35 g per litre and the upper limit is, independently, 0.60, 0.50 or 0.45 g per litre. For example, a solution for use according to the invention may comprise 0.40 g acesulfame K per litre. In this embodiment, sucralose is present in an amount of 0.2 to 1.0 g per litre, preferably within a range in which the lower limit is 0.2 or 0.4 g per litre and the upper limit is, independently, 1.0, 0.8, 0.6, 0.5, 0.45, 0.3 g per litre. For example, a solution for use according to the invention may comprise 0.6 g or 0.45 g sucralose per litre.

Thus, in one embodiment of the present invention, the solution for use according to the invention comprises 0.6g or thereabout sucralose per litre and 0.4g or thereabout acesulfame K per litre. In another embodiment, the solution for use according to the invention comprises 0.45g or thereabout sucralose per litre and 0.4g or thereabout acesulfame K per litre. Preferably, sucralose and acesulfame K are present in a weight ratio of sucralose : acesulfame K of from 2:1 to 1:2, more preferably from 1.25:1 to 1:1.

### Flavouring

Solutions for use according to the invention optionally comprise one or more flavourings. Flavourings assist in making the solutions for ingestion more palatable to certain patients. The exact level of flavouring required depends on the intensity of flavour desired, and the nature and strength of the flavour in question. Typically, a flavouring may be present at a level of 1 to 10g per litre, for example from 1 to 5g per litre, especially from 1.2 to 4.3g per litre, such as 1.5 to 3.7g per litre, for example 1.5g, 2.0g, 2.5g, 3.2g, 3.7g or 4.0g per litre.

Examples of flavours that can be used include orange, lemon-lime, lemon, citrus, chocolate, tropical fruit, aloe vera, tea, strawberry, grapefruit, blackcurrant, pineapple and vanilla. Preferred flavours are orange juice flavour, lemon and lime and tropical fruit flavour. Citrus flavour may also be used.

Those and further suitable flavourings are available from various flavour manufacturers and suppliers, for example International Flavours and Fragrances Inc. (Duddery Hill, Haverhill, Suffolk, CB9 8LG, England), Ungerer & Company (Sealand Road, Chester, England CH1 4LP), Firmenich (Firmenich UK Ltd., Hayes Road, Southall, Middlesex UB2 5NN) or S. Black Ltd (Foxholes Business Park, John Tate Road, Hertford, Herts, SG13 7YH, United Kingdom). Solutions for use according to the invention are preferably substantially free from added citrate ions. Citrate ions are provided for example by citric acid and sodium citrate. Some fruit flavourings may intrinsically contain a small amount of citric acid. Those amounts are not considered substantial in this context. Solutions for use according to the invention are preferably substantially free from added acid. Hydrogen ions are provided for example by organic acids (for example citric acid or ascorbic acid) or inorganic acids (for example hydrochloric acid). Some fruit flavourings may intrinsically contain small amounts of organic acids. Those amounts are not considered substantial in this context. Solutions for use according to the invention preferably have a pH of 8.0 to 11.0, preferably 8.0 to 10.5, for example 8.4 to 9.0. Measurements of pH may, for example, be carried out with a Hanna Instruments "pH ep" temperature compensating pH meter.

In another embodiment of the invention, an aqueous solution for use according to the invention has the following components at the following concentrations:
(a) 350 to 600 g/L PEG having an average molecular weight of 2500 to 4500 Da;
(b) 8.0 to 20 g/L sodium chloride;
(c) 1.0 to 3.0 g/L potassium chloride;
(d) 3.0 to 11 g/L sodium bicarbonate;
(f) optional flavouring; and
(g) optional sweetener.

In all respects other than in relation to the added preservative component, the solution has the preferred features described elsewhere herein. Containers and kits comprising such solutions, methods of preparing such solutions are also provided.

The invention further provides a unit treatment of a solution for use according to the invention, the unit treatment having the volume necessary to provide 11 to 15g of PEG. An alternative unit treatment of a solution for use according to the invention has the volume necessary to provide 5.5 to 7.5g of PEG (for use in mild cases of constipation or the paediatric population).

For example, a unit treatment may be from 10 to 50ml of the solution for use according to the invention. For example, if a solution for use according to the invention comprises 525 g of PEG 3350 per litre, then 25ml are required to provide the amount of PEG shown above. A unit treatment is thus preferably from 20 to 40ml, for example 25 to 30ml, especially 25 ml. Accordingly, the invention further provides a unit treatment of from 10 to 50ml of the solution for use according to the invention. Preferably, a unit treatment is from 20 to 40ml, for example 25 to 30ml, especially 25 ml. For use in an alternative setting (for example for paediatric use or in patients with mild constipation) mentioned above, all of the quantities in a unit treatment are halved.

Also described herein, but not claimed, is a container containing a solution described herein. For example, a container may contain:
(a) x x 262.50g polyethylene glycol (PEG) 3350;
(b) x x 7.014 g sodium chloride;
(c) x x 0.932g potassium chloride;
(d) x x 3.570g sodium bicarbonate;
(e) optionally x x an amount of preservative;
(f) optionally x x an amount of flavouring;
(g) optionally x x an amount of sweetener; and
(h) water to x x 500ml;
where x is 0.5 to 2, for example x is 0.5 or 1.

Also described herein, but not claimed, is a container containing:
(a) *y* x 13.125g polyethylene glycol (PEG) 3350;
(b) *y* x 0.3507g sodium chloride;
(c) *y* x 0.0466g potassium chloride;
(d) *y* x 0.1785g sodium bicarbonate;
(e) optionally *y* x an amount of preservative;
(f) optionally *y* x an amount of flavouring;
(g) optionally *y* x an amount of sweetener; and
(h) water to *y* x 25 ml;
where *y* is 0.5 to 40, for example *y* is 0.5 or 1 or 2 or 4 or 10.

Such a container may, for example, contain:
(a) 262.50g PEG 3350;
(b) 7.014 g sodium chloride;
(c) 0.932g potassium chloride;
(d) 3.570g sodium bicarbonate;
(e) optional preservative (but where present in an amount of from 1.5 to 2.95g) ;
(f) optional flavouring (but where present in an amount of from 0.5 to 5.0 g);
(g) optional sweetener (but where present in an amount of from 0.05 to 0.75g); and
(h) water to 500ml.

An alternative container may contain:
(a) 131.25g PEG 3350;
(b) 3.507g sodium chloride;
(c) 0.466g potassium chloride;
(d) 1.785g sodium bicarbonate;
(e) optional preservative (but where present in an amount of from 0.75 to 1.475g) ;
(f) optional flavouring (but where present in an amount of from 0.25 to 2.5g);
(g) optional sweetener (but where present in an amount of from 0.025 to 0.375g); and
(h) water to 250ml.

An alternative container may contain:
(a) 78.75g PEG 3350;
(b) 2.1042 g sodium chloride;
(c) 0.2796g potassium chloride;
(d) 1.071g sodium bicarbonate;
(e) optional preservative (but where present in an amount of from 0.45 to 0.88g);
(f) optional flavouring (but where present in an amount of from 0.15 to 1.5g);
(g) optional sweetener (but where present in an amount of from 0.015 to 0.225g); and
(h) water to 150ml.

An alternative container may contain:
(a) 52.5g PEG 3350;
(b) 1.4028g sodium chloride;
(c) 0.1864g potassium chloride;
(d) 0.714g sodium bicarbonate;
(e) optional preservative (but where present in an amount of from 0.3 to 0.59g);
(f) optional flavouring (but where present in an amount of from 0.1 to 1.0g);
(g) optional sweetener (but where present in an amount of from 0.01 to 0.15g); and
(h) water to 100ml.

An alternative container may contain:
(a) 13.125g PEG 3350;
(b) 0.3507g sodium chloride;
(c) 0.0466g potassium chloride;
(d) 0.1785g sodium bicarbonate;
(e) optional preservative (but where present in an amount of from 0.075 to 0.1475g);
(f) optional flavouring (but where present in an amount of from 0.025 to 0.25g);
(g) optional sweetener (but where present in an amount of from 0.0025 to 0.0375 g); and
(h) water to 25ml.

The solutions for use according to the present invention, optionally presented in a container comprising single or multiple treatment units, are preferably provided with instructions for use. The invention further provides a kit comprising a container containing a solution for use according to the invention together with instructions to consume a unit amount of the invention. The instructions may also state what disorders (such as constipation or faecal impaction), the solution of the invention is indicated for. The invention further provides a kit comprising a container containing a solution for use according to the invention together with a measuring accessory for measuring out a defined volume of the solution. Examples of measuring accessories include measuring cups, measuring spoons, measuring tubes, and syringes. If 25ml is the unit measurement, the measuring accessory preferably enables the measurement of a 25ml unit treatment out of the bottle or container.

The measuring accessory may be adapted to attach to the container, for example it may be in the form of a cap that fits over and grips onto the closure of the container in storage and can be held separately from the container for measuring out a required volume of solution. The measuring accessory may have a measurement volume such that several measurement accessories provide the required unit treatment volume. For example, for the provision of a 25ml unit treatment volume, a measuring accessory might provide for measuring a volume of 25ml, 12.5ml (two needed), 8.333ml (three needed), 6.25ml (4 needed) or 5ml (5 needed). A suitable measuring accessory may have the required volume as its total capacity, or it may be provided with one or more gradation lines to indicate the required volume. In one embodiment, the measuring accessory is a cap that provides for the measurement of a 25ml unit volume. For use in an alternative setting (for example for paediatric use or in patients with mild constipation) mentioned above, the volumes mentioned here are all halved. For distribution and sale, a container may be provided in an outer packaging, such as a carton. Instructions may be provided on a medium, for example paper, inside the outer packaging. Instructions may be printed onto the outer packaging, or onto the container itself. A carton may contain the container, a measuring accessory and instructions. Alternatively or additionally, instructions may direct the user to a website where information such as background information on approved indications, recommended dose, composition of the solution, frequency of recommended use, and the like are displayed.

Solutions for use according to the invention are suitable for use in the treatment of constipation and/or faecal impaction. Accordingly, the solutions for use according to the present invention find use in a method of treating constipation or faecal impaction comprising administering orally, without further dilution, to a subject an effective amount of the solution of the invention. The solutions for use according to the invention find use as a medicament; for example the medicament can be for use in the treatment of constipation or faecal impaction. A solution for use in a method described herein has the preferred features described above in respect of the solutions for use according to the invention.

The solution for use according to the invention may be provided in a container having a volume that is for multiple unit treatments. The solution for use according to the invention may therefore be provided in a container that contains sufficient solution for any convenient number of unit treatments. For example, the container might provide 1, 2, 4, 5, 8, 10, 12, 15, 20, 25, 30, 35, 40, 50, 60, 70, 75, 80 or 100 unit treatments. For example, if a solution for use according to the invention comprises 525 g of PEG 3350 per litre, and 25ml are required to provide the 13.125g of PEG in a standard dose, then a container may provide 25ml, 50ml, 100ml, 125ml, 150ml, 200ml, 250ml, 300ml, 375ml, 500ml, 625ml, 750ml, 875ml or 11 of solution. For example a container may contain one or more unit treatments of solution, each unit treatment having a volume of 20 to 50ml, for example 25ml, or each unit treatment having a volume of 7.5 to 25ml, for example 12.5ml. For example, a container (for example a bottle) of the invention provides 100ml, 150ml, 250ml or 500ml of solution of the invention.

Suitable containers include bottles, for example with a re-closable closure. A re-closable closure may be child-proof. A re-closable closure may be tamper-evident. Containers may for example be made of plastic or glass, for example polyethylene terephthalate (PET). They may be circular in cross-section, for example they may be a right circular cylinder. They may be transparent, translucent or opaque; containers may be coloured, for example amber.

In another embodiment of the invention, the solution for use according to the invention is provided as a single unit amount which may be particularly convenient when access to safe drinkable water is not readily available, for instance, when the user is travelling. This embodiment is also more readily portable and avoids the need to carry more units than are needed. The single unit amount of solution for use according to the invention may be disposed within a container of suitable, preferably minimal, volume such as a bottle, pouch, stickpack, sachet or can. Since this embodiment encompasses a single dose unit amount of solution for use according to the invention, a preservative may not be needed in order to achieve an acceptable shelf-life. A plurality of single unit dose containers may be in a box, for example to provide a course of treatment. It is preferred that packaging used, particularly those parts in direct contact with the aqueous solution of PEG, are substantially free (preferably entirely free) of aluminium.

Also described herein, but not claimed, is a container such as a bottle, pouch, sachet, stickpack or can comprising a single unit amount of the following components;
(a) 13.125g PEG 3350;
(b) 0.3507g sodium chloride;
(c) 0.0466g potassium chloride;
(d) 0.1785g sodium bicarbonate;
(e) optional preservative (but where present in an amount of from 0.075 to 0.1475g);
(f) optional flavouring (but where present in an amount of from 0.025 to 0.25g);
(g) optional sweetener (but where present in an amount of from 0.0025 to 0.0375 g); and
(h) water to 25ml.

The following solutions in Table 1 (Tables 1a to 1c) are disclosed herein. These solutions are specifically disclaimed as *per se* solutions in this application. Solutions specifically disclosed in international patent application PCT/GB2010/001455 are specifically disclaimed from *per se* solution claims in this application.

**Table 1a: Common components of the solutions in Tables 1b and 1c**

| **Component** | **Quantity/g** |
|---|---|
| PEG 3350 | 13.1250 |
| Sodium Chloride | 0.3507 |
| Potassium Chloride | 0.0466 |
| Sodium Bicarbonate | 0.1785 |
| Acesulfame K | 0.0100 |
| Optional Preservative | As indicated |
| Optional Flavour | As indicated |
| Optional Additional Sweetener | As indicated |
| Water | As indicated |

In Tables 1b and 1c, the flavour "TF" is Tropical Fruit and the flavor "OJ" is Orange Juice. They are available from Firmenich UK Ltd (Hayes Road, Southall, Middlesex, UB2 5NN).

**Table 1b:**

| **Component** | **2A^{§}** | **2B^{§}** | **2C^{§}** | **2D^{§}** | **2E^{§}** | **2F^{§}** | **2G^{§}** | **2H^{§}** |
|---|---|---|---|---|---|---|---|---|
| Flavour | None | None | 0.0375gTF | 0.0375gTF | 0.0375gTF | 0.0375gTF | 0.0375gTF | 0.0375gTF |
| Sucralose | None | None | 0.0150g | 0.0150g | 0.0150g | 0.0150g | 0.0150g | 0.0150g |
| Sodium propyl paraben (sodium propyl 4-hydroxybenzoate) | 0.05% [0.01625g] | 0.1% [0.0325g] | | | | | | |
| Blend of methyl paraben (18%), ethyl paraben (9%) and benzyl alcohol (73%) | | | 0.3% [0.075g] | 0.5% [0.125g] | 0.7% [0.175g] | | | |
| Phenoxy ethanol | | | | | | 0.7% [0.175g] | | |
| Methyl paraben (as sodium salt) | | | | | | | 0.15% [0.0375g] | |
| Ethyl paraben (as sodium salt) | | | | | | | 0.10% [0.025g] | |
| Blend of methyl paraben (18%), ethyl paraben (9%) and phenoxyethanol (73%) | | | | | | | | 0.5% [0.125g] |
| Water | to 32.5ml | to 32.5ml | to 25ml | to 25ml | to 25ml | to 25ml | to 25ml | to 25ml |

| | | | | | | | | |
|---|---|---|---|---|---|---|---|---|
| § Solution not within the scope of the claims. | | | | | | | | |

**Table 1c:**

| **Component** | **2I^{§}** | **2J^{§}** | **2K^{§}** | **2L^{§}** | **2M^{§}** | **2N^{§}** | **2P^{§}** | **2Q^{§}** |
|---|---|---|---|---|---|---|---|---|
| Flavour | 0.0375gTF | 0.0375gTF | 0.0800gOJ | 0.0800gOJ | 0.0800gOJ | 0.0800gOJ | 0.0800gOJ | 0.0800gOJ |
| Sucralose | 0.0150g | 0.0150g | 0.003g | 0.003g | 0.003g | 0.003g | 0.003g | 0.003g |
| Methyl paraben | | | 0.18% [0.045g] | 0.225% [0.05625g] | 0.0675% [0.0169g] | | 0.0675% [0.0169g] | |
| Propyl paraben | | | 0.02% [0.005g] | 0.025% [0.00625g] | | | | |
| Ethyl paraben | | | | | | 0.0338% [0.0084g] | | 0.0338% [0.0084g] |
| Benzyl alcohol | | | | | | | 0.1825% [0.0456g] | 0.2163% [0.05406g] |
| Propylene glycol | | | 3.75g | 3.75g | | | | |
| Blend of methyl paraben (15%), ethyl paraben (10%) and phenoxyethanol (75%) | 0.5% [0.125g] | 0.8% [0.200g] | | | | | | |
| Water | to 25ml | to 25ml | to 25ml | to 25ml | to 25ml | to 25ml | to 25ml | to 25ml |

| | | | | | | | | |
|---|---|---|---|---|---|---|---|---|
| § Solution not within the scope of the claims. | | | | | | | | |

### Examples

### Example 1: Solutions of the invention

Solutions 1A to IN were prepared. The solutions contained the components shown in Table 2, and each solution was made up with water to a total volume of 25 ml.

**Table 2:**

| **Solution No.** | **Flavouring** | **Macrogol 3350 (g)** | **Sodium Chloride (g)** | **Sodium Bicarbonate (g)** | **Potassium Chloride (g)** | **Acesulfame K (g)** | **Sucralose (g)** | **Flavour (g)** | **Preservative type and (g)** |
|---|---|---|---|---|---|---|---|---|---|
| 1A^{§} | Lemon-Lime | 13.125 | 0.3507 | 0.1785 | 0.0466 | 0.0100 | 0.0112 | 0.0925 | Iscaguard MEB 0.0875g |
| 1B^{§} | Tropical | 13.125 | 0.3507 | 0.1785 | 0.0466 | 0.0100 | 0.0112 | 0.0375 | Iscaguard MEB 0.1000g |
| 1C^{§} | Orange | 13.125 | 0.3507 | 0.1785 | 0.0466 | 0.0100 | 0.0112 | 0.0800 | Iscaguard MEB 0.0875g |
| 1D^{§} | Orange | 13.125 | 0.3507 | 0.1785 | 0.0466 | 0.0100 | 0.0112 | 0.0800 | Iscaguard MEB 0.1000g |
| 1E^{§} | Lemon-Lime | 13.125 | 0.3507 | 0.1785 | 0.0466 | 0.0100 | 0.0112 | 0.0625 | Iscaguard MEB 0.0875g |
| 1F^{§} | Lemon-Lime | 13.125 | 0.3507 | 0.1785 | 0.0466 | 0.0100 | 0.0112 | 0.0500 | Iscaguard MEB 0.0875g |
| 1G^{§} | Tropical | 13.125 | 0.3507 | 0.1785 | 0.0466 | 0.0100 | 0.0112 | 0.0375 | Iscaguard MEB 0.1000g |
| 1H^{§} | Tropical | 13.125 | 0.3507 | 0.1785 | 0.0466 | 0.0100 | 0.0112 | 0.1000 | Iscaguard MEB 0.1000g |
| 1I^{§} | Lemon-Lime | 13.125 | 0.3507 | 0.1785 | 0.0466 | 0.0100 | 0.0112 | 0.0925 | Methyl Paraben 0.0200g Ethyl Paraben 0.0100g |

**Table 2 continued:**

| **Solution No.** | **Flavouring** | **Macrogol 3350 (g)** | **Sodium Chloride (g)** | **Sodium Bicarbonate (g)** | **Potassium Chloride (g)** | **Acesulfame K (g)** | **Sucralose (g)** | **Flavour (g)** | **Preservative type and (g)** |
|---|---|---|---|---|---|---|---|---|---|
| 1J^{§} | Lemon-Lime | 13.125 | 0.3507 | 0.1785 | 0.0466 | 0.0100 | 0.0112 | 0.0925 | Methyl Paraben 0.0250g Ethyl Paraben 0.0130g |
| 1K^{§} | Tropical | 13.125 | 0.3507 | 0.1785 | 0.0466 | 0.0100 | 0.0112 | 0.0375 | Methyl Paraben 0.0200g Ethyl Paraben 0.0100g |
| 1L^{§} | Tropical | 13.125 | 0.3507 | 0.1785 | 0.0466 | 0.0100 | 0.0112 | 0.0375 | Methyl Paraben 0.0250g Ethyl Paraben 0.0130g |
| 1M^{§} | Lemon Lime | 13.125 | 0.3507 | 0.1785 | 0.0466 | 0.0100 | 0.0112 | 0.0925 | Iscaguard MEB 0.0875g Propyl Paraben 0.025g |
| 1N^{§} | Lemon Lime | 13.125 | 0.3507 | 0.1785 | 0.0466 | 0.0100 | 0.0112 | 0.0925 | Iscaguard MEB 0.0875g Propyl Paraben 0.050g |

| | | | | | | | | | |
|---|---|---|---|---|---|---|---|---|---|
| § Solution not within the scope of the claims. | | | | | | | | | |

### Example 2: Efficacy of antimicrobial preservative.

Five 20g portions of each sample were transferred to sterile glass bottles and inoculated separately with 0.2mL culture of the test species as detailed below. The test species used include the following shown in Table 3.

**Table 3:**

| |
|---|
| Species |
| Pseudomonas aeruginosa |
| Escherichia coli |
| Staphylococcus aureus |
| Candida albicans |
| Aspergillus brasiliensis |

The inoculated sample portions were mixed using sterile implements and stored at room temperature. The challenge test protocol as set forth in 5.1.3 of the European Pharmacopoeia, version 7.2 was then followed.

Results:
Samples of formulation 1A and 1B were tested for efficacy of the preservative at time (T) T=4 weeks, T=9 weeks and T=12 weeks and passed the acceptance criteria (Table 5.1.3.3, European Pharmacopoeia, 7.2) at all measured time points. Specifically, the combined CFU count for yeast and mould was determined to be less than 10 CFU/ml at all measured time points.

### Example 3: Container containing a solution described herein

Bottle 2a: A 500ml polyethylene terephthalate (PET) bottle contains 500ml of a solution 1A to IN described in Example 1 above. The bottle is provided with a re-closable closure, and a cap that fits over and grips onto the closure. The cap is suitable for measuring a 25ml unit treatment volume. The bottle is provided in a carton with instructions for use. The instructions include the instruction that 25ml of the solution should be measured out and taken neat as a treatment dose.

Bottle 2b is a 250ml polyethylene terephthalate (PET) bottle containing 250ml of a solution 1A to IN described in Example 1 above.

Bottles 2c and 2d are the same as bottles 2a and 2b respectively, but with a cap that is suitable for measuring a 12.5ml unit treatment volume, and instructions that include instruction that 12.5ml of the solution should be measured out and taken neat as a treatment dose.

Pouch 2e contains 25ml of a solution 1A to IN described in Example 1 above. Single use container 2f contains 25ml of a solution 1A to IN described in Example 1 above. Stickpack 2g contains 25ml of a solution 1A to IN described in Example 1 above. Each of pouch 2e, single use container 2f and stickpack 2g is provided with instructions for use. The instructions include the instruction that 25ml of the solution should be taken neat as a treatment dose. A plurality of pouches 2e, single use containers 2f or stickpacks 2g are provided in a carton.

## Claims

1. An aqueous solution single unit dose amount, consisting essentially of the following components:
(a) 350 to 600g/L PEG having an average molecular weight of 2500 to 4500 Da;
(b) 8.0 to 20 g/L sodium chloride;
(c) 1.0 to 3.0 g/L potassium chloride;
(d) 3.0 to 11 g/L sodium bicarbonate;
(e) optional sweetener; and
(f) optional flavouring,
the solution being substantially free from added preservative,
for use in a method of treating constipation and/or faecal impaction in a mammalian subject, such as a human, comprising orally administering the solution without further dilution.

2. The solution for use according to claim 1 wherein sodium chloride is within a range wherein the lower limit is 10, 11, 12 or 13g per litre and the upper limit is, independently, 18, 17, 16 or 15g per litre such as 13 to 15g per litre, e.g. 14.028g per litre.

3. The solution for use according to claim 1 or claim 2 wherein the potassium chloride is in an amount of 1.0 to 3.0g per litre, preferably in a range wherein the lower limit is 1.2, 1.4, 1.6, 1.7 or 1.8g per litre and the upper limit is, independently 2.7, 2.5, 2.3, 2.1 or 2.0g per litre, e.g. 1.8g to 1.9g per litre.

4. The solution for use according to any preceding claim wherein the sodium bicarbonate is in an amount of 3.0 to 11g per litre, preferably within a range wherein the lower limit is 5.0, 6.0, 6.5 or 7.0g per litre and the upper limit is, independently, 10, 9.0, 8.0 or 7.5g per litre; for example 6.5 to 8.0 g per litre; for example approximately 7.1 g per litre, for example 7.140g per litre.

5. The solution for use according to any preceding claim comprising 0.1 to 1.5 g/L of sweetener selected from the group consisting of; aspartame, acesulfame potassium, sucralose, saccharine and combinations thereof.

6. The solution for use according to any preceding claim comprising 1.0 to 10.0g/L of flavouring, for example from 1 to 5g per litre, especially from 1.2 to 4.3g per litre, such as 1.5 to 3.7g per litre, for example 1.5g, 2.0g, 2.5g, 3.2g, 3.7g or 4.0g per litre.

7. The solution for use according to any preceding claim comprising PEG (which is preferably PEG3350 or PEG4000) in a range wherein the lower limit is 400, 450 or 500 g per litre and the upper limit is, independently, 600, 575 or 550 g per litre; for example 500 to 550 g per litre.

8. A kit comprising:
(A) an aqueous solution, preferably substantially free of sulphate, consisting essentially of:
(a) 350 to 600g/L PEG (or a sub-range thereof such as 500 to 550g/L) having an average molecular weight of 2500 to 4500 Da (particularly 3350),
the solution being substantially free from added preservative; and
(B) a composition in a solid form such as a capsule or tablet (e.g. film coated tablet) comprising;
(b) sodium chloride in a range of 0.1 to 3.0g, preferably, 0.3g. to 1.5g, even more preferably, 0.3g to 1.0g, e.g. 0.3g to 0.4g;
(c) potassium chloride in a range of 0.01g to 0.1g, preferably, 0.3g to 0.6g, more preferably, 0.4g to 0.5g;
(d) sodium bicarbonate, in the range of 0.1g to 0.3g, preferably, 0.15g to 0.2g, more preferably, 0.17g to 0.18g.

9. The kit of claim 8 wherein the aqueous solution and/or solid form further comprise a sweetener, the sweetener for example being selected from the group consisting of; aspartame, acesulfame potassium, sucralose, saccharine, sodium cyclamate and combinations thereof.

10. The kit of claim 8 or 9 wherein the aqueous solution and/or solid form further comprises a flavouring (such as strawberry, lemon-lime, tropical fruit or orange).

11. The kit of claim 10 wherein the flavouring is present in the aqueous solution at a concentration of 1 to 10g per litre, for example from 1 to 5g per litre, especially from 1.2 to 4.3g per litre, such as 1.5 to 3.7g per litre, for example 1.5g, 2.0g, 2.5g, 3.2g, 3.7g or 4.0g per litre.

12. A kit as claimed in any one of claims 8 to 11 for use as a medicament.

13. A kit for use as claimed in claim 12 for use in the treatment of constipation and/or faecal impaction.

14. A kit for use as claimed in claim 13 for use in the treatment of constipation and/or faecal impaction by oral administration without further dilution of the solution component.

15. An aqueous solution for use according to claim 1, wherein said use comprises the steps of;
(a) administering or consuming the aqueous solution, and;
(b) administering or consuming a second aqueous solution.

## Patentansprüche

1. Eine wässrige Lösung in Einzeleinheitsdosismenge, bestehend im Wesentlichen aus den folgenden Komponenten:
(a) 350 bis 600 g/l PEG mit einem mittleren Molekulargewicht von 2500 bis 4500 Da;
(b) 8,0 bis 20 g/l Natriumchlorid;
(c) 1,0 bis 3,0 g/l Kaliumchlorid;
(d) 3,0 bis 11 g/l Natriumbicarbonat;
(e) optionales Süßungsmittel und
(f) optionaler Aromastoff,
wobei die Lösung im Wesentlichen frei von zugegebenem Konservierungsmittel ist,
zur Verwendung bei einem Verfahren zur Behandlung von Obstipation und/oder Impaktbildung bei einem Subjekt, das ein Säuger ist, wie z.B. ein Mensch, umfassend die orale Verabreichung der Lösung ohne weitere Verdünnung.

2. Die Lösung zur Verwendung gemäß Anspruch 1, wobei Natriumchlorid in einem Bereich, bei dem die Untergrenze 10, 11, 12 oder 13 g pro Liter ist und die Obergrenze unabhängig davon 18, 17, 16 oder 15 g pro Liter ist, wie z.B. 13 bis 15 g pro Liter, z.B. 14,028 g pro Liter, vorliegt.

3. Die Lösung zur Verwendung gemäß Anspruch 1 oder Anspruch 2, wobei das Kaliumchlorid in einer Menge von 1,0 bis 3,0 g pro Liter, vorzugweise in einem Bereich, bei dem die Untergrenze 1,2, 1,4, 1,6, 1,7 oder 1,8 g pro Liter ist und die Obergrenze unabhängig davon 2,7, 2,5, 2,3, 2,1 oder 2,0 g pro Liter ist, z.B. 1,8 g bis 1,9 g pro Liter, vorliegt.

4. Die Lösung zur Verwendung gemäß einem vorhergehenden Anspruch, wobei das Natriumbicarbonat in einer Menge von 3,0 bis 11 g pro Liter, vorzugsweise in einem Bereich, bei dem die Untergrenze 5,0, 6,0, 6,5 oder 7,0 g pro Liter ist und die Obergrenze unabhängig davon 10, 9,0, 8,0 oder 7,5 g pro Liter ist, zum Beispiel 6,5 bis 8,0 g pro Liter, zum Beispiel etwa 7,1 g pro Liter, zum Beispiel 7,140 g pro Liter, vorliegt.

5. Die Lösung zur Verwendung gemäß einem vorhergehenden Anspruch, umfassend 0,1 bis 1,5 g/l Süßungsmittel, ausgewählt aus der Gruppe bestehend aus Aspartam, Acesulfam-Kalium, Sucralose, Saccharin und Kombinationen davon.

6. Die Lösung zur Verwendung gemäß einem vorhergehenden Anspruch, umfassend 1,0 bis 10,0 g/l Aromastoff, zum Beispiel 1 bis 5 g pro Liter, insbesondere 1,2 bis 4,3 g pro Liter, wie z.B. 1,5 bis 3,7 g pro Liter, zum Beispiel 1,5 g, 2,0 g, 2,5 g, 3,2 g, 3,7 g oder 4,0 g pro Liter.

7. Die Lösung zur Verwendung gemäß einem vorhergehenden Anspruch, umfassend PEG (das vorzugsweise PEG3350 oder PEG4000 ist) in einem Bereich, bei dem die Untergrenze 400, 450 oder 500 g pro Liter ist und die Obergrenze unabhängig davon 600, 575 oder 550 g pro Liter ist, zum Beispiel 500 bis 550 g pro Liter.

8. Ein Kit, umfassend:
(A) eine wässrige Lösung, die vorzugsweise im Wesentlichen frei von Sulfat ist, bestehend im Wesentlichen aus:
(a) 350 bis 600 g/l PEG (oder ein Teilbereich davon, wie z.B. 500 bis 550 g/l) mit einem mittleren Molekulargewicht von 2500 bis 4500 Da (speziell 3350),
wobei die Lösung im Wesentlichen frei von zugegebenem Konservierungsmittel ist; und
(B) eine Zusammensetzung in einer festen Form, wie z.B. einer Kapsel oder Tablette (z.B. eine filmbeschichtete Tablette), umfassend:
(b) Natriumchlorid in einem Bereich von 0,1 bis 3,0 g, vorzugsweise 0,3 g bis 1,5 g, besonders bevorzugt 0,3 g bis 1,0 g, z.B. 0,3 g bis 0,4 g;
(c) Kaliumchlorid in einem Bereich von 0,01 g bis 0,1 g, vorzugsweise 0,3 g bis 0,6 g, besonders bevorzugt 0,4 g bis 0,5 g;
(d) Natriumbicarbonat im Bereich von 0,1 g bis 0,3 g, vorzugsweise 0,15 g bis 0,2 g, besonders bevorzugt 0,17 g bis 0,18 g.

9. Das Kit nach Anspruch 8, wobei die wässrige Lösung und/oder die feste Form ferner ein Süßungsmittel umfassen/umfasst, wobei das Süßungsmittel zum Beispiel ausgewählt ist aus der Gruppe bestehend aus Aspartam, Acesulfam-Kalium, Sucralose, Saccharin, Natriumcyclamat und Kombinationen davon.

10. Das Kit nach Anspruch 8 oder 9, wobei die wässrige Lösung und/oder die feste Form ferner einen Aromastoff (wie z.B. Erdbeere, Zitrone-Limette, Südfrüchte oder Orange) umfassen/umfasst.

11. Das Kit nach Anspruch 10, wobei der Aromastoff in der wässrigen Lösung in einer Konzentration von 1 bis 10 g pro Liter, zum Beispiel von 1 bis 5 g pro Liter, insbesondere von 1,2 bis 4,3 g pro Liter, wie z.B. 1,5 bis 3,7 g pro Liter, zum Beispiel 1,5 g, 2,0 g, 2,5 g, 3,2 g, 3,7 g oder 4,0 g pro Liter, vorliegt.

12. Ein Kit wie in einem der Ansprüche 8 bis 11 beansprucht zur Verwendung als Medikament.

13. Ein Kit zur Verwendung wie in Anspruch 12 beansprucht zur Verwendung bei der Behandlung von Obstipation und/oder Impaktbildung.

14. Ein Kit zur Verwendung wie in Anspruch 13 beansprucht zur Verwendung bei der Behandlung von Obstipation und/oder Impaktbildung durch orale Verabreichung ohne weitere Verdünnung der Lösungskomponente.

15. Eine wässrige Lösung zur Verwendung gemäß Anspruch 1, wobei die Verwendung die folgenden Schritte umfasst:
(a) Verabreichen oder Konsumieren der wässrigen Lösung und
(b) Verabreichen oder Konsumieren einer zweiten wässrigen Lösung.

## Revendications

1. Une solution aqueuse à dose unitaire unique, composée essentiellement des composants suivants:
(a) 350 à 600g/L PEG ayant un poids moléculaire moyen de 2500 à 4500 Da;
(b) 8,0 à 20 g/L de chlorure de sodium;
(c) 1,0 à 3,0 g/L de chlorure de potassium;
d) 3,0 à 11 g/L de bicarbonate de sodium;
e) édulcorant facultatif; et
f) arôme facultatif;
la solution étant substantiellement exempte d'agent de conservation à titre d'ajout,
pour une utilisation dans une méthode de traitement de la constipation et/ou de l'impaction fécale chez un sujet mammifère, tel qu'un humain, comprenant l'administration orale de la solution sans dilution supplémentaire.

2. La solution à utiliser selon la revendication 1, dans laquelle le chlorure de sodium se situe dans une plage dans laquelle la limite inférieure est de 10, 11, 12 ou 13g par litre et la limite supérieure est, indépendamment, de 18, 17, 16 ou 15g par litre tel que 13 à 15g par litre, par exemple 14,028g par litre.

3. La solution à utiliser selon la revendication 1 ou la revendication 2, dans laquelle le chlorure de potassium est en une quantité de 1,0 à 3,0 g par litre, de préférence dans une plage dont la limite inférieure est de 1,2, 1,4, 1,6, 1,7 ou 1,8 g par litre et dont la limite supérieure est, indépendamment, de 2,7, 2,5, 2,3, 2,1 ou 2,0 g par litre, par exemple 1,8 g à 1,9 g par litre.

4. La solution à utiliser selon l'une quelconque des revendications précédentes, dans laquelle le bicarbonate de sodium est en une quantité de 3,0 à 11g par litre, de préférence dans une plage dans laquelle la limite inférieure est de 30 5,0, 6,0, 6,5 ou 7,0 g par litre et la limite supérieure est, indépendamment, de 10, 9,0, 8,0 ou 7,5 g par litre; par exemple 6,5 à 8,0 g par litre; par exemple environ 7,1 g par litre, par exemple 7,140g par litre.

5. La solution à utiliser selon l'une quelconque des revendications précédentes, comprenant de 0,1 à 1,5 g/L d'édulcorant choisi dans le groupe constitué par l'aspartame, l'acésulfame de potassium, le sucralose, la saccharine et leurs combinaisons.

6. La solution à utiliser selon l'une quelconque des revendications précédentes, comprenant de 1,0 à 10,0 g/L d'arôme, par exemple de 1 à 5 g par litre, en particulier de 1,2 à 4,3 g par litre, tels que 1,5 à 3,7 g par litre, par exemple 1,5 g, 2,0 g, 2,5 g, 3,2 g, 3,7 g ou 4,0 g par litre.

7. La solution à utiliser selon l'une quelconque des revendications précédentes, comprenant du PEG (qui est de préférence PEG3350 ou PEG4000) dans une plage dont la limite inférieure est de 400, 450 ou 500 g par litre et la limite supérieure est, indépendamment, de 600, 575 ou 550 g par litre; par exemple 500 à 550 g par litre.

8. Une trousse ou kit comprenant :
A) une solution aqueuse, de préférence substantiellement exempte de sulfate, consistant essentiellement en:
a) 350 à 600 g/L de PEG (ou une sous-gamme de celui-ci de 500 à 550 g/L) ayant un poids moléculaire moyen de 2500 à 4500 Da (en particulier 3350);
la solution étant substantiellement exempte d'agent de conservation à titre d'ajout; et
(B) une composition sous forme solide telle qu'une capsule ou un comprimé (par exemple un comprimé pelliculé) comprenant;
(b) du chlorure de sodium dans une plage de 0,1 à 3,0 g, de préférence, 0,3g à 1,5 g, encore plus préférentiellement, 0,3 g à 1,0 g, par exemple 0,3 g à 0,4g;
(c) du chlorure de potassium dans une plage de 0,01 g à 0. 1 g, de préférence, 0,3g à 0,6g, plus préférentiellement, 0,4g à 0,5g;
d) du bicarbonate de sodium, dans une plage de 0,1g à 0,3 g, de préférence, 0,15 g à 0,2 g, plus préférentiellement, 0,17 g à 0,18 g.

9. La trousse ou kit selon la revendication 8, dans laquelle la solution aqueuse et/ou la forme solide comprend en outre un édulcorant, l'édulcorant étant par exemple choisi dans le groupe constitué par: aspartame, acésulfame de potassium, sucralose, saccharine, cyclamate de sodium et leurs combinaisons.

10. La trousse ou kit selon la revendication 8 ou 9, dans laquelle la solution aqueuse et/ou la forme solide comprend en outre un arôme (tel que fraise, citron-lime, fruit tropical ou orange).

11. La trousse ou kit selon la revendication 10, dans lequel l'arôme est présent dans la solution aqueuse à une concentration de 1 à 10g par litre, par exemple de 1 à 5 g par litre, en particulier de 1,2 à 4,3 g par litre, comme 1,5 à 3,7 g par litre, par exemple 1,5 g, 2,0 g, 2,5 g, 3,2 g, 3,7 g ou 4.0 g par litre.

12. La trousse ou kit tel que revendiqué dans l'une des revendications 8 à 11, pour une utilisation comme médicament.

13. La trousse ou kit à utiliser comme indiqué dans la revendication 12, pour une utilisation dans le traitement de la constipation et/ou de l'impaction fécale.

14. La trousse ou kit destiné à être utilisé tel que revendiqué dans la revendication 13, pour une utilisation dans le traitement de la constipation et/ou de l'impaction fécale par administration orale sans dilution supplémentaire du composant de la solution.

15. Une solution aqueuse destinée à être utilisée selon la revendication 1, dans laquelle ladite utilisation comprend les étapes de;
(a) administration ou consommation de la solution aqueuse, et;
(b) administration ou consommation d'une deuxième solution aqueuse.
